Europäisches Patentamt

European Patent Office

Office européen des brevets

(11) Publication number: **0 604 693 A1**

# EUROPEAN PATENT APPLICATION

(21) Application number: **92403571.0**

(22) Date of filing: **29.12.92**

(51) Int. Cl.5: **C12P 21/08**, C07K 15/28, C07K 13/00, C12N 5/20, C07K 3/18, G01N 33/577, //C12N15/13

(43) Date of publication of application:
**06.07.94 Bulletin 94/27**

(84) Designated Contracting States:
**FR**

(71) Applicant: **SCHERING-PLOUGH**
**92, rue Baudin**
**F-92307 Levallois-Perret Cédex(FR)**

(72) Inventor: **Galizzi, Jean-Pierre**
**Résidence Melerose,**
**36 Chemin de la Pomme**
**F-69160 Tassin la Demi Lune(FR)**
Inventor: **Diossou, Odile**
**Chemin du Gareizin**
**F-69340 Francheville(FR)**
Inventor: **Banchereau, Jacques**
**"La Boissiére",**
**25 Avenue Paul Santy**
**F-69130 Ecully(FR)**

(74) Representative: **Durand, Yves Armand Louis**
**Cabinet Z. Weinstein**
**20, Avenue de Friedland**
**F-75008 Paris (FR)**

(54) Monoclonal antibodies against the human interleukin-4 receptor and hybridomas producing the same.

(57) The present invention provides compounds and compositions useful for the detection, purification, measurement and/or inhibition of the human 130 kDa IL-4 receptor. The compounds and compositions are derived from hybridomas producing monoclonal antibodies specific for the human 130 kDa IL-4 receptor. In particular, the invention includes hybridomas selected from the group consisting of S103, 0361, S17, 0296, S456, S924, S697, 0497 and 0735 and their monoclonal antibodies and products derived therefrom. The invention also includes methods of using the above compounds and compositions to detect, purify, and measure the concentration of human 130 kDa IL-4, and kits for practicing such methods; and pharmaceutical compositions useful in treating IL-4 related illnesses.

Rank Xerox (UK) Business Services
(3.10/3.09/3.3.4)

BACKGROUND OF THE INVENTION

Interleukin-4 is a pleiotropic cytokine involved in numerous aspects of the immune response. It has T cell growth factor (TCGF) activity, and B cell growth factor activity. It is capable of potentiating the TCGF activity of interleukin-2 (IL-2) and the colony-forming activity of granulocyte-macrophage colony stimulating factor (GM-CSF). It induces the preferential production of IgG$_1$ and IgE, induces the low affinity receptor for IgE (CD23), and induces the expression of human leukocyte class II DR antigens.

The IgE- and CD23-inducing activity of IL-4 could have important consequences for persons suffering from allergic diseases. The availablility of IL-4 antagonists could provide an alternative to the use of glucocorticoid steroids, which have many deleterious side effects, especially with prolonged usage.

IL-4 binds to high affinity receptors which include a 130 kDa glycoprotein [Galizzi *et al.*, "Purification of a 130-kDa T cell glycoprotein that binds human interleukin 4 with high affinity", J. Biol. Chem. Vol. 265: page 439; (1990a), and Galizzi *et al.*, "Molecular cloning of a cDNA encoding the human interleukin-4 receptor", Int. Immunol. Vol. 2: page 669 (1990b)].

Crosslinking studies with labelled IL-4 indicate the presence of 80, 70 kDa IL-4 binding molecules in addition to the 130 kDa component [Galizzi *et al.*, "Internalization of human interleukin-4 and transient down-regulation of its receptor in the CD23-inducible Jijoye cells", J. Sol. Chem. Vol. 264: page 6984 (1989)]. Antibodies specific for the IL-4 receptor which block the binding of IL-4 would permit one to inhibit IL-4 biological effects. As IL-4 plays a key role in the production of IgE such blocking antibodies could be therapeutic entities for allergy. Non-human monoclonal antibodies could advantageously be humanized [Winter, G., and Milstein, C., Man-made antibodies. Nature. Vol. 349 : page 293 (1991)] and thus be used for long term treatment of allergic disorders. Antibodies specific to the human 130 kDa IL-4 receptor may also block the development of cytotoxic T cells and therefore be used to prevent the rejection of grafts.

A combination of antibodies should permit the establishment of an ELISA assay allowing the detection of soluble IL-4R in human biological fluids. This will permit one to establish whether increasing or decreasing circulating levels of IL-4 receptor is of diagnostic value in allergy or graft rejection.

Such antibodies specific for the 130 kDa IL-4R will permit one to determine whether other proteins exist which bind IL-4 with high affinity (Galizzi, 1989 *supra*).

SUMMARY OF THE INVENTION

The present invention provides compounds and compositions useful for the detection, purification, measurement and/or inhibition of the human 130 kDa IL-4 receptor.

The present invention also provides compounds and compositions that are useful for the treatement of IL-4-related diseases.

The compounds and compositions are derived from hybridomas producing monoclonal antibodies specific for the human 130 kDa IL-4 receptor. The compounds and compositions of the invention include the hybridomas themselves, monoclonal antibodies produced by the hybridomas, heavy chain and light chain variable region polypeptides and other fragments of such antibodies such as half-molecules comprising a light chain joined to a heavy chain by natural disulfide bonds, Fab fragments, F(ab)$_2$ fragments, Fv fragments, single chain Fv, and the like.

The invention also includes methods of using the above compounds and compositions to detect, purify, and measure the concentration of human 130 kDa IL-4, and kits for practicing such methods. In particular, the invention includes hybridomas selected from the group consisting of S103, 0361, S17, 0296, S456, S924, S697, 0497 and 0735 and their monoclonal antibodies and products derived therefrom.

The present invention still further provides for isolated DNAs which encode heavy or light chain variable regions of a monoclonal antibody produced from the above-mentioned hybridomas which specifically bind to the human 130 kDa IL-4 receptor or complementarity determining regions (CDRs) of such antibody, or functional equivalents thereof.

The invention still further provides binding compositions, single-chain binding proteins, and chimeric or humanized monoclonal antibodies comprising CDRs from the light and/or heavy chain variable regions of the monoclonal antibodies of the above-mentioned hybridomas.

Pharmaceutical compositions comprising a human IL-4 antagonist selected from the group consisting of monoclonal antibodies produced from the above-mentioned hybridomas, a binding composition which specfically binds to the human 130 kDa interleukin-4 receptor comprising a heavy chain variable region (V$_H$) and a light chain variable region (V$_L$) from a monoclonal antibody produced by one of the above-mentioned hybridomas, a single-chain binding protein which specifically binds to the human 130 kDa IL-4 receptor comprising CDRs from the light and/or heavy chain variable regions of a monoclonal antibody produced by one of the above-mentioned hybridomas, a chimeric monoclonal antibody which specfically binds to the human 130 kDa IL-4 receptor comprising the heavy and light chain variable regions of a monoclonal antibody produced by one of the above-mentioned hybridomas, and a humanized monoclonal antibody which specifically binds to the human 130 kDa IL-4 receptor comprising CDRs from the heavy and light chain variable regions of a monoclonal antibody produced by one of the above-mentioned hybridomas; and a physiologically acceptable carrier, are also provided by the present invention.

BRIEF DESCRIPTION OF THE FIGURES

**Figures 1 A-D show** inhibition of IL-4 induced biological effects by IL-4R specific mAbs.

**A**/PBMC were preactivated for 4 days with 1 $\mu$g/ml PHA and viable blasts were recultured for 3 days with 50 pM IL-4 and increasing concentrations of S103, S456, S924. Cells were pulsed with ($^3$H)dThd for the last 16 h.

**B**/ Purified tonsil **B** cells were cultured for 3 days with 5 mg/ml of insolubilized anti-IgM plus 50 pM IL-4 in the presence of increasing concentrations of purified S103, S456 and S924.

**C and D**/ Purified tonsil B cells were cultured for 48 h with a combination of 2.5 pM IL-4 and S103, S456, S924. Cells were stained for CD23 expression (C) or IgM expression (D) using specific anti-CD23 or anti-IgM antibodies and FITC- conjugated anti-mouse IgG. Florescence staining was analyzed with the FACScan.

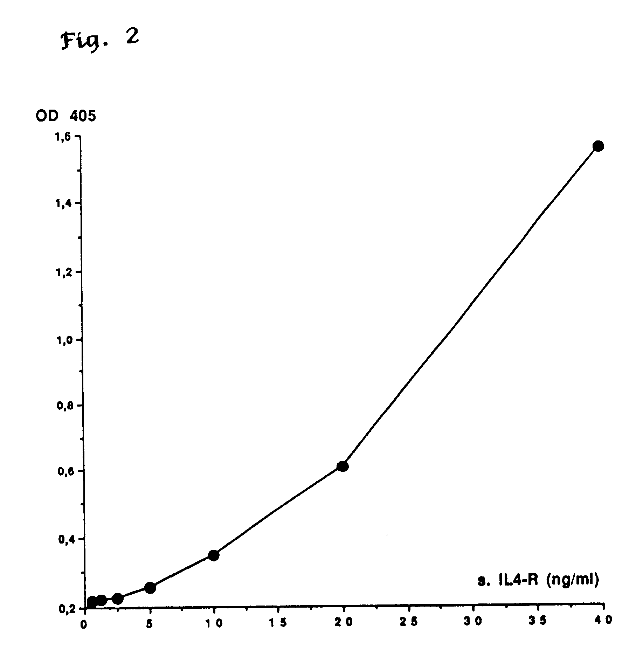

**Figure 2** shows sandwich ELISA for soluble IL-4R.

Plates were coated with a mixture of mAb (S17, 0296, 0361, 0497) and increasing concentrations of soluble IL-4R were added. A second biotinylated antibody (S456) was then incubated with the preformed complex and S456 binding revealed by sequential addition of alkaline phosphatase conjugated streptavidin and the enzymatic substrate.

DETAILED DESCRIPTION OF THE INVENTION

All references cited herein are hereby incorporated in their entirety by reference.

As used herein, the terms "DNA" and "DNAs" are defined as molecules comprising deoxyribonucleotides linked in standard 5' to 3' phosphodiester linkage, including both smaller oligodeoxyribonucleotides and larger deoxyribonucleic acids.

Antibodies comprise an assembly of polypeptide chains linked together by disulfide bridges. Two principal polypeptide chains, referred to as the light chain and the heavy chain, make up all major structural classes (isotypes) of antibody. Both heavy chains and light chains are further divided into subregions referred to as variable regions and constant regions. Heavy chains comprise a variable region ($V_H$) and three or four different constant regions, and light chains comprise a single variable region ($V_L$) different from that of the heavy chain and a constant region different from that of the heavy chain.

As used herein, the term "heavy chain variable region" means a polypeptide (1) which is from 110 to 125 amino acids in length, and (2) whose amino acid sequence corresponds to that of a heavy chain of a monoclonal antibody of the invention, starting from the heavy chain's N-terminal amino acid. Likewise, the term "light chain variable region" means a polypeptide (1) which is from 95 to 115 amino acids in length, and (2) whose amino acid sequence corresponds to that of a light chain of a monoclonal antibody of the invention, starting from the light chain's N-terminal amino acid.

The variable regions of the heavy chain and light chain are responsible for the antibody's binding specficity. The variable regions of both the heavy and light chains are comprised of three hypervariable regions referred to as Complementarity Determining Regions (CDR) which are located near positions 30, 50 and 95. It is generally accepted that such hypervariable regions are involved directly in the formation of the antigen-binding site. In between the CDRs in both the light and heavy chains are four Framework Regions (FRs) which possess less variability than the CDRs.

As used herein, the term "binding composition means a composition comprising two polypeptide chains (1) which, when operationally associated, assume a conformation having high binding affinity for the human 130 kDa IL-4 receptor; and (2) which are derived from a hybridoma producing monoclonal antibodies to the human 130 kDa IL-4 receptor. The term "operationally associated" is meant to indicate that the two polypeptide chains can be positioned relative to one another for binding by a variety of means, including association in a native antibody fragment, such as Fab or Fv, or by way of genetically engineered peptide linkers.

Hybridomas of the invention are produced by well-known techniques. Usually, the process involves the fusion of an immortalizing cell line with a B-lymphocyte that produces the desired antibody. Alternatively, non-fusion techniques for generating immortal antibody producing cell lines are possible, and come within the purview of the present invention, e.g. virally induced transformation: Casali *et al.*, "Human Monoclonals from Antigen-Specific Selection of B Lymphocytes and Transformation by EBV", Science, Vol. 234: pgs. 476-479(1986). Immortalized cell-lines are usually transformed mammalian cells, particularly myeloma cells of rodent, bovine, and human origin. Most frequently, rat or mouse myeloma cell-lines are employed as a matter of convenience and availability.

Techniques for obtaining the appropriate lymphocytes for mammals injected with the target antigen are well known. Generally, peripheral blood lymphocytes (PBLs) are used if cells of human origin are desired, or spleen cells or lymph node cells are used if non-human mammalian sources are desired. A host mammal is injected with repeated dosages of the purified antigen, and the mammal is permitted to generate the desired antibody producing cells before these are harvested for fusion with the immortalizing cell-line. Techniques for fusion are also well known in the art, and in general involve mixing the cells with a fusing agent, such as polyethylene glycol. Hybridomas are selected by standard procedures, such as HAT selection. From among these hybridomas, those secreting the desired antibody are selected by assaying their culture medium by standard immunoassays, such as Western blotting, ELISA, RIA or the like. Antibodies are recovered from the medium using standard protein-purification techniques. Many references are available for guidance in applying any of the above techniques: e.g. Kohler *et al.*, Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques are Applications (CRC Press, Boca Raton, FL, 1982); and the like.

The use and generation of fragments of antibodies are also well known; e.g. Fab fragments: Tijssen, *supra*; and Fv fragments: Hochman *et al.* Biochemistry, Vol. 12: pgs. 1130-1135(1973), Sharon *et al.*, Biochemistry, Vol. 15: pgs 1591-1594(1976) and Ehrlich *et al.*, U.S. Patent 4,355,023; and antibody half molecules: Auditore-Hargreaves, U.S. Patent 4,470,925. Moreover, such compounds and compositions of the invention can be used to construct bi-specific antibodies by known techniques; e.g., by further fusions of hybridomas (i.e. to form so-called quadromas): Reading, U.S. Patent 4,474,493; or by chemical reassociation of half molecules: Brennan *et al.*, Science, Vol. 229: pgs. 81-83 (1985). Also known are single-chain Fv polypeptides which incorporates the complete antibody binding site in a single polypeptide chain. These can be produced by connecting the heavy chain variable region ($V_H$) of an antibody with the light chain variable region ($V_L$) by means of a peptide linker. See PCT International Publication Nos. WO 88/09344, WO 92/15682 and U.S. Patent No. 5,019,513.

Hybridomas and monoclonal antibodies of the invention are produced against either glycosylated or unglycosylated versions of the recombinantly-produced mature human 130 kDa IL-4 receptor. Generally, unglycosylated versions of the human 130 kDa IL-4 receptor are produced in *E. coli*, and glycosylated versions are produced in mammalian cell hosts, e.g. CV1 or COS monkey cells, mouse L cells, or the like. Recombinantly produced mature human IL-4 receptor is produced by introducing an expression vector into a host cell using standard protocols; e.g. Maniatis *et al.*, Molecular Cloning: A Laboratory Manual (Cold Spring Harbor Laboratory, New York, 1982); Okayama and Berg, Mol. Cell. Biol., Vol. 2: pgs. 161-170(1982) and Vol. 3: pgs. 280-289 (1983); Hamer, Genetic Engineering , Vol. 2: pgs. 83-100 (1980) and U.S. Patent 4,599,308; Kaufman *et al.*, Mol. Cell. Biol., Vol. 2: pgs. 1304-1319 (1982); or the like.

Construction of bacterial or mammalian expression vectors is well known in the art, once the nucleotide sequence encoding a desired protein is known or otherwise available; e.g. DeBoer in U.S. Patent 4,511,433 discloses promoters for use in bacterial expression vectors; Goeddel *et al.*, in U.S. Patent 4,601,980, and Riggs, in U.S. Patent 4,431,739, disclose the production of mammalian proteins by *E. coli* expression systems; and Riggs *supra*, Ferretti *et al.*, Proc. Natl. Acad. Sci., Vol. 83: pgs. 599-603 (1986), Sproat *et al.*, Nucleic Acids Research, Vol. 13: pgs. 2959-2977 (1985), and Mullenbach *et al.*, J. Biol. Chem.. Vol. 261: pgs. 719-722 (1986), disclose how to construct synthetic genes for expression in bacteria. Accordingly, these references are incorporated by reference. The amino acid sequence of the mature 130 kDa human IL-4 receptor and the cDNA encoding the human 130 kDa IL-4 receptor carried by the pME 18S vector have been disclosed by Gallizzi *et al.*, "Molecular cloning of a cDNA encoding the human interleukin-4 receptor". Int. Immunol. Vol. 2: page 669(1990). *E. coli* MC1061 carrying pME 18S-hIL-4R containing the cDNA encoding the human 130 kDa IL-4 receptor has been deposited on March 20th 1990 with the American Type Culture Collection, Rockville, Maryland, USA (ATCC) and has accession number 68263.

Many bacterial expression vectors and hosts are available commercially or through the ATCC. Preferably, the human 130 kDa IL-4 for immunizing host animals is isolated from culture supernatants of COS, CV1, or mouse L cells which have been transiently transfected by the above-mentioned pME 18S-hIL-4R vector.

In particular, such techniques can be used to produce interspecies monoclonal antibodies, wherein the binding region of one species is combined with a non-binding region of the antibody of another species. [Liu *et al.*, Proc. Natl. Acad. Sci. USA, Vol 84: page 3439(1987)]. For example, the CDRs from a rodent monoclonal antibody can be grafted onto a human antibody, thereby "humanizing" the rodent antibody [Riechmann *et al.*, Nature Vol. 332: page 323 (1988)]. More particularly, the CDRs can be grafted into a human antibody variable region with or without human constant regions. Such methodology has been used to humanize a mouse monoclonal antibody against the p55 (Tac) subunit of the human interleukin-2 receptor [Queen *et al.*, Proc. Natl. Acad. Sci. USA Vol. 86: page 10029 (1989)].

Messenger RNA (mRNA) extracted from the hybridomas of the invention is useful for cloning and expressing fragments of the monoclonal antibodies in bacteria, yeast, or other hosts. Complementary DNAs (cDNA) produced from such mRNA which encode the heavy and light chain variable regions and CDRs of such monoclonal antibodies can be used to produce engineered antibodies and single-chain binding proteins by standard methods.

The location of the CDRs within the variable regions of the antibodies can be determined using a number of well known standard methods. For example, Kabat *et al.*, [*Sequences of Proteins of Immunological Interest* 4th Edition, 1987, U.S. Department of Health and Human Services, National Institutes of Health] have published rules for locating CDRs. Computer programs are also available which can be used to identify CDRs on the basis of the amino acid residues involved in the three-dimensional binding site loops of the antibody chains.

If the CDRs from the antibodies of the present invention are used to produce humanized antibodies by grafting onto a human antibody, it may be desirable to include one or more amino acid residues which, while outside the CDRs, are likely to interact with the CDRs or the human 130 kDa IL-4 receptor. (Queen *et al., supra*).

The CDRs of the invention can also form the basis for the design of non-peptide mimetic compounds which mimic the functional properties of antibodies of the present invention. Methods for producing such mimetic compounds have been described by Saragovi *et al.*, Science Vol. 253: page 792(1991).

Antibodies and antibody fragments characteristic of hybridomas of the invention can also be produced by recombinant means by extracting messenger RNA, constructing a cDNA library, and selecting clones which encode segments of the antibody molecule: e.g. Wall *et al.*, Nucleic Acids Research, Vol. 5: pgs. 3113-3128(1978); Zalsut *et al.*, Nucleic Acids Research, Vol. 8: pgs. 3591-3601(1980); Cabilly *et al.*, Proc. Natl. Acad. Sci., Vol. 81: pgs. 3273-3277(1984); Boss *et al.*, Nucleic Acids Research, Vol. 12: pgs. 3791-3806(1984); Amster *et al.*, Nucleic Acids Research, Vol. 8: pgs. 2055-2065(1980); and Moore *et al.*, U.S. Patent 4,642,234. In particular, such techniques can be used to produce interspecies monoclonal antibodies, wherein the binding region of one species is combined with a non-binding region of the antibody of another species; e.g. Liu *et al.*, Proc. Natl. Acad. Sci., Vol. 84, pgs. 3439-3443 (1987).

Uses of monoclonal antibodies for purification and measurement are well known, e.g. affinity chromatography: Affinity Chromatography: Principles and Methods (Pharmacia, Orebro, Sweden, 1979); Secher *et al.*, Nature, Vol. 285: pgs. 446-450 (1980), and U.S. Patent 4,423,147; and European patent application 0190711 (13 August 1986); and immunoassay techniques: Tijssen *supra*; U.S. Patent 4,486,530; and Burnette *supra*. Affinity chromatography can be used to purify the human 130 kDa IL-4 receptor by extracting it from a sample, such as a culture supernatant of cells transformed or transfected with a human IL-4 receptor expression vector. Such a purification process is referred to herein as an immunopurification process. Typically, it involves covalently attaching a monoclonal antibody specific for the human 130 kDa IL-4 receptor to a solid phase support (referred to herein as an "immunoadsorbent") which is placed in a column or chamber through which the sample is passed. The human 130 kDa IL-4 receptor from the sample preferentially binds to the binding sites of the attached monoclonal antibodies, while the rest of the material from the sample is washed from the column or chamber. The human 130 kDa IL-4 receptor is then eluted from the immunoadsorbent by standard techniques, e.g. low pH, high salt concentration, or the like.

"Two site" or "sandwich" immunoassays are the preferred immunoassays of the invention, e.g. as disclosed in U.S. Patent 4,486,530. Accordingly, this patent is incorporated by reference. Such assays entail the use of two different sets of anti-IL-4 receptor antibodies, at least one of which consists of a monoclonal antibody of the invention. Antibodies from one of the two sets are attached to the surface of a solid-phase support. The attached antibodies are then exposed to a sample suspected of containing the human 130 kDa IL-4 receptor. The IL-4 receptor molecules bind to the attached antibodies. Next, the second set of antibodies is applied to the bound IL-4 receptor, and these bind to one or more antigenic determinants distinct from that (or those) to which the first set of antibodies is bound. The IL-4 receptor is then detected by an indirect or direct signal-generating means associated with the second set of antibodies. For example, the antibodies can be directly conjugated to a signal-generating moiety, such as an enzyme, rare earth chelator, or an organic dye. Or they can be indirectly linked to one or more signal-generating moieties through additional antibodies, or high affinity complexes, such as the avidin-biotin complexes. Quantitative measurements of IL-4 receptor concentration are made by comparing the signal generated by the sample with signals generated by IL-4 receptor standards containing known concentrations of the human IL-4 receptor.

The invention includes kits of reagents for use in immunoassays, particularly sandwich immunoassays. Such kits include (1) a solid-phase support, (2) a first antibody which is monoclonal and is capable of binding to a first antigenic determinant of the human IL-4 receptor, (3) a second antibody selected from the group consisting of a monoclonal antibody capable of binding to a second antigenic determinant of the human IL-4 receptor and a polyclonal antibody specific for the human IL-4 receptor (referred to herein as a "polyclonal antibody composition"), and (4) a signal-generation means associated with one of the three antibodies. Depending on the particular embodiment, kits may include a selection of two of the three anti-IL-4 receptor antibody types, either a monoclonal antibody specific for a first antigenic determinant and a monoclonal antibody specific for a second antigenic determinant, or a monoclonal antibody specific for a first or second antigenic determinant and a polyclonal antibody composition. The antibodies may be in solution or in lyophilized form. One of the sets of antibodies may be available for application to the surface of the solid support when the kit is used, or may come pre-attached to the solid support. The signal-generating means may come pre-associated with one of the two antibody types, or may require combination with one or more components, e.g. buffers, antibody-enzyme conjugates, enzyme substrates, or the like, prior to use. Many types of signal-generating means are available and can make up one or more components of a kit. Various signal-generating means are disclosed by Tijssen *supra*. Kits of the invention may also include additional reagents, e.g. blocking reagents for reducing nonspecific binding to the solid-phase surface, washing reagents, enzyme substrates, and the like. The solid-phase surface may be in the form of microtiter plates, microspheres, or the like, composed of polyvinyl chloride, polystyrene, or the like materials suitable for immobilizing proteins. Such materials having solid-phase surfaces are referred to herein as "support means". Preferably, an enzyme which catalyzes the formation of a fluorescent or colored product is a component of the signal-generating means. More preferably, the enzyme is selected from the group consisting of peroxidase, alkaline phosphatase, and beta-galactosidase. Substrates and reaction conditions for these enzymes are well known in the art; e.g., Tijssen *supra*.

Compositions of the invention can also be used as components of pharmaceutical compositions directed against IL-4-related diseases. Pharmaceutical compositions that comprise monoclonal antibodies (or binding fragments thereof or single chain Fvs) having blocking or antagonistic effects can be used to suppress IL-4 activity by binding to the IL-4 receptor instead of IL-4 binding to the receptor. Such compositions contain a therapeutic amount of at least one of the monoclonal antibodies of the invention, or binding fragments or single-chain Fvs thereof, in a pharmaceutically effective carrier. A pharmaceutical carrier can be any compatible, non-toxic substance suitable for delivering the compositions of the invention to a patient. Sterile water, alcohol, fats, waxes, and inert solids may be included in a carrier. Pharmaceutically acceptable adjuvants (buffering agents, dispersing agents) may also be incorporated into the pharmaceutical composition. Generally, compositions useful for parenteral administration of such drugs are well known; e.g. Remington's Pharmaceutical Science, 15th Ed. (Mack Publishing Company, Easton, PA, 1980). Alternatively, compositions of the invention may be introduced into a patient's body by implantable drug delivery systems; e.g. Urquhart *et al.*, Ann. Rev. Pharmacol.Toxicol., Vol. 24: pgs. 199-236 (1984).

EXAMPLES

The following examples serve to illustrate the present invention. Selection of vectors and hosts as well as the concentration of reagents, temperatures, and the values of other variables are only to exemplify application of the present invention and are not to be considered limitations thereof.

EXAMPLE 1

PRODUCTION OF SOLUBLE IL-4 RECEPTOR

● **Cell culture and reagents.**

Soluble human 130 kDa IL-4R was derived from Cos 7 cells transfected with a plasmid containing a cDNA encoding the extracellular domain of the human 130 kDa IL-4R. This cDNA was obtained by *Dra III* digestion of the full length IL-4R (Galizzi *et al. supra*, 1990b and PCT publication number WO 91/03555) and encoded for a 200 amino acid protein. *E. coli* MC1061 carrying pME 18S-hIL-4R containing the cDNA encoding the human 130 kDa IL-4 receptor has been deposited on March 20th 1990 with the American Type Culture Collection, Rockville, Maryland, USA (ATCC) and has accession number 68263.

The recombinant protein was purified from transfected cell culture supernatant by an IL-4-Affi-Gel 10 chromatography as described by Galizzi *et al. supra* 1990a). Purified phytohemaglutin (PHA) was from Wellcome (Dartford, G.B.). Fluorescein isothiocyanate (FITC) conjugated F(ab')$_2$ fragment of goat anti-mouse Ig were from Grub (Vienna, Austria). Phycoerythrine conjugated streptavidin (Streptavidin-PE) was from Becton-Dickinson (Mountain View, CA). Jijoye cells, a Burkitt lymphoma and L cells, a fibroblast cell line, were from ATCC. All cell lines and tonsil B cells were cultured in complete culture medium RPMI 1640 (Gibco) supplemented with 10% fetal calf serum (FCS), 2 mM glutamine, 50 $\mu$g/ml gentamycin (Schering-Plough). G418 was from Gibco.

### ● Transfection and fluorescence activated sorting of L cells.

The neo-resistant gene was introduced into a mammalian expression vector, pME18S, containing the human 130 kDa IL-4R cDNA. L cells, plated at $10^6$ cells/10cm plate the previous day, were harvested by trypsinisation and washed once with RPMI 1640, 10% FCS and resuspended at $11 \times 10^6$ cells/ml in RPMI 1640. 25 $\mu$g of linearized (KPNI endonuclease) plasmid (pME18S neo IL-4R or pME18S neo) DNA were added to 0.7 ml of cell suspension ($10 \times 10^6$ cells/ml) in a 0.4 cm electroporation cuvette (Bio-Rad, Richmond, CA). Electroporation was performed using the Gene pulser (Bio-Rad, Richmond, CA) at 960 $\mu$F and 250 V. Transfectants were selected with G418 (1 mg/ml). After one week selection $40 \times 10^6$ cells, transfected with IL-4R, were harvested in phosphate buffered saline (PBS), 0.5 mM EDTA, washed by centrifugation (150 g, 5 min) and the pellet resuspended at $10 \times 10^6$ cells/ml in PBS, 0.5 mM EDTA, 0.1% bovine serum albumin (BSA) (PEB buffer). Cell suspension was incubated (4°C) with $10^{-7}$ M biotinylated IL-4 in the absence or presence (non-specific) of $4 \times 10^{-6}$ M cold IL-4. After 90 min incubation at 4°C, cells were washed twice by centrifugation with PEB buffer, and further incubated with 1/5 dilution of streptavidin-phycoerythrine for 30 min at 4°C ($10 \times 10^6$ cells/ml). Then, samples were washed twice by centrifugation and resuspended at $10^6$ cells in PEB. Cells were sorted on a FACS 440 (Becton-Dickinson) equipped with a 5-W argon laser running at 488 nm, 0.5 W. The 4% brightest stained cells were collected at each sort and cultured in 1 mg/ml G418 containing culture medium until the next sort. After 4 cycles of sorting, cells were cloned at 0.5 cell per well (96 well-plates). One clone (C70), selected among 300, expressed 500000 sites/cells, as measured by $^{125}$I-IL-4 binding.

### EXAMPLE 2

### PRODUCTION OF ANTIBODIES TO THE IL-4 RECEPTOR

### ● Immunization of mice and fusion.

4 BALB/C mice (Iffa Credo, Les Oncins, France) were immunized intraperitoneally with 10 $\mu$g soluble hIL-4R in Freund's complete adjuvant. At three week intervals, booster immunizations ($2 \times 10$ $\mu$g, soluble hIL-4R) were given in Freund's incomplete adjuvant. Following 3 injections, significant $^{125}$I-IL-4 binding inhibition to Jijoye cells and staining of the hIL-4R transfected L cell were noted in immune mouse sera, at a 1/1000 dilution, in comparison to normal mouse sera. Immune mouse spleen cells ($300 \times 10^6$ cells) were fused with NS1 myeloma cells (ratio 5 : 1) using 50% polyethylene glycol, 1500 (Boehringer, Mannheim) in 50 mM HEPES pH 7. Twenty 96 well plates were seeded with $2 \times 10^5$ spleen B cells/well (100 $\mu$l) in a RPMI 1640 medium containing 0.1 mM hypoxanthine, 0.058 mM azaserine, 0.15/ml oxaloacetate, 0.05 mg/ml pyruvate, 0.2 UI/ml insuline, 10% hybridoma cloning efficiency factor (EGS, Interchim), 20% FCS, 2 mM L-glutamine, and 0.04 mg/ml gentamicine. Cells were fed on day 6 by adding 50 $\mu$l of the above medium. Hybridomas secreting antibodies specific for IL-4R were screened at day 7 for their ability to stain L cells transfected with the human IL-4R. Hybridomas secreting antibodies against IL-4R were cultured in 48 well plates, rescreened and recloned by limiting dilution until 100% of the 96 wells were positive. Mab isotypes was determined with the mouse typer isotyping kit (Bio-Rad, Richmond, CA).

### ● Screening of hybridoma supernatants.

$10^6$ L cells transfected with pME18s neoIL-4R(C70)or the pME18s neo,were incubated 30 min at 4°C with 50 $\mu$l of hybridoma supernatant. After 2 washes by centrifugation with PBS, 1% BSA, 0.1% azide cells were further incubated with FITC conjugated goat anti-mouse IgG (1/1000 dilution) for 30 min at 4°C. After 2 washes, cells were resuspended in PBS, 1% BSA, 0.1% azide and propidium iodide (5 $\mu$g/ml) to help in gating out non-viable cells and then analyzed with the FACScan. Among the 1900 hybridoma supernatants, 47 stained C70 but not the control non-transfected L cells.

● **Ascites production and antibody purification.**

For ascite production, BALB/C mice were injected intraperitoneally with 0.5 ml of pristane (Sigma, Saint Louis, Mo). One week later, $2 \times 10^6$ hybridoma cells were injected intraperitoneally and ascitic fluid was collected 2 to 3 weeks later. The ascitic fluid was incubated 1 h at 37°C, 2 h at 4°C and centrifuged at 1000 g, 15 min, to remove lipids and cellular debris. 1 ml ascites fluid was purified on an anion exchange (DEAE 5 PW, Waters Associates, Millipore Corp., Milford, MA) equilibrated with Tris-Cl 50 mM, pH8. Antibodies were eluted using NaCl gradient (3.75 mM NaCl/min; buffer 1 M NaCl, Tris-Cl pH8) at a flow rate of 8 ml/min. Purity of the Mabs was assessed by SDS PAGE (10% acrylamide) analysis according to Laemmli (Laemmli, 1970) and silver stained. Before electrophoresis, samples were adjusted to 2% SDS, 10% glycerol, 0.01% bromophenol blue and 5%-$2\beta$ mercaptoethanol.

EXAMPLE 3

● **Inhibition of $^{125}$I-IL-4 binding to cells.**

Purified human rIL-4 ($10^7$ U/mg), produced in *E. coli*, was obtained from Schering-Plough Research (Bloomfield, N.J). IL-4 was labelled with $^{125}$I Na using a Tejedor-Ballesta method at the specific radioactivity of 2000-4000 cpm/formol, as described above. Binding on cell lines and PHA blasts was performed as described (Galizzi, 1990a *supra*). $3 \times 10^6$ cells were incubated in RPMI 1640, 1% BSA at 4°C in the presence of increasing concentrations of sera, hybridoma supernatant or purified antibodies for 30 min. Then, 50 pM of $^{125}$I-IL-4 was added with or without 10 mM cold IL-4. After 4 h, samples (500 $\mu$l total) were washed twice by centrifugation (150 gx 3 min) with 2 ml binding medium at 4°C and radioactivity of the cell pellet was determined in a counter.

EXAMPLE 4

● **Biotinylation and cross-competition between Mab for binding to cell lines.**

1 mg of antibody was biotinylated as described by Bayer *et al.* "The use of avidin-biotin complex as a tool in molecular biology". Methods Biochem. Vol. 26: page 1(1980). $5 \times 10^5$ L cells (C70) were incubated with unlabeled antibody in PBS, 1% BSA, 0.1% azide (PBA), 15 min at 4°C. Cells were washed with 200 $\mu$l PBA (x2) and stained with 200 ng of the biotinylated anti-IL-4R antibodies for 15 min. After washing twice, cells were incubated with 100 $\mu$l (1/5 dilution in PBA) of streptavidin phycoerythrine, washed (x2) and analyzed by FACS.

EXAMPLE 5

● **Immunoprecipitation and Western blot analysis.**

1/Immunoprecipitation :$^{125}$I-labelled soluble IL-4R were prepared at the specific radioactivity of 3300 Ci/mmol by incubating 1 $\mu$g of soluble IL-4R with 0.5 mCi $^{125}$I Na and 25 $\mu$l of chloramin T (10 $\mu$g/mg protein) in 150 $\mu$l PBS for 5 min. Then, free $^{125}$I was separated from the bound by PD10 gel permeation (Pharmacia). 10 $\mu$g of anti-mouse IgG (Sigma) in 100 $\mu$l of PBS were coated on polyvinyl microplates (96 wells, Dynatech) at 4°C, overnight. Then the plates were saturated 1 h at 25°C with 300 $\mu$l, 10% Fetal Calf Serum (FCS) PBS. After washing with PBS (4x), wells were filled with 100 $\mu$l (10 $\mu$g/ml) of anti-IL-4R antibodies, 4 h at 4°C. Then wells were incubated with 3 nM soluble $^{125}$I-IL-4R in PBS 10% Fetal Calf Serum (FCS) (100 $\mu$l). After 12 h, wells were washed and radioactivity extracted with SDS gel buffer and the cpm were counted. Samples were also analyzed by electrophoresis to confirm the presence of soluble IL-4R
2/ Western blot analysis: 50 $\mu$l of purified soluble IL-4R (20 pmoles) were run on SDS-PAGE at 4°C under non reducing conditions. Proteins were electrotransferred from SDS gel onto nitrocellulose membranes (Hybond-C super, Amersham) at 200 mA (1h), as previously described (Galizzi, 1990a *supra*). The membranes were incubated 1 h with 8 M urea, 0.1 % Tween, 50 mM HEPES-NaOH (pH 7.4), washed 3 times with TNT buffer (100 mM Tris-Cl pM 7.5,140 mM NaCl, 0.1% Tween), and blocked 1 h with 5% nonfat dry milk in TNT buffer. For ligand blotting (purified sIL-4R), membranes were incubated 3 h at 4°C with $3\times10^6$ cpm/ml, $^{125}$I-IL-4 (0.4 nM) with or without 20 nM cold IL-4 washed with TNT buffer, dried and autoradiographed. For Western blotting (Cos 7 supernatant, Triton extract), membranes were incubated at 4°C, 1 h, with 10 $\mu$g/ml of monoclonal anti-IL-4R antibody in 50 mM Tris-HCl pH 7.5, 150 mM NaCl, washed 4 times with TNT and incubated 1 h with Horseradish peroxidase labelled anti-mouse IgG. Then, anti-IL-4R binding to nitrocellulose was revealed by chemiluminescence detection using ECL kit (Amesham) according to the manufacturer's recommended protocol and based on the oxidation of luminol by peroxide.

## EXAMPLE 6

### ● ELISA assay

96 well microplates were coated with 100 $\mu$l of S17, 0296, 0361 and 0497 mAb (1 $\mu$g/ml in coating buffer 0.1 H sodium carbonate pH 9.6) and blocked with 1% BSA in PBS. Purified soluble IL-4R was added to each well at various concentrations and incubated 2 h at 25°C. The wells were washed with 0.05 Tween 20 in PBS and incubated 2 h at 25°C with 100 $\mu$l of biotinylated mAb (S546) (1/1000 dilution of 1 mg/ml S456). The bound biotinylated S456 was detected by sequential incubation with alkaline phosphatase labelled streptavidin and the enzyme substrate solution.

## EXAMPLE 7

### ● Proliferation of PHA blasts.

PBMC were isolated from healthy donors by centrifugation on standard Ficoll/Hypaque (Pharmacia, Uppsala, Sweden) density gradient. Cells were cultured at $1\times10^6$ cells/ml with 1 $\mu$g/ml PHA in complete culture medium which consisted of RPMI 1640 supplemented with 10% heat-inactivated FCS, 2 mM L-glutamine, 100 U/ml penicillin, 100 $\mu$g/ml streptomycin (all from Flow Laboratories, Irvine, Scotland), 50 $\mu$g/ml gentamyciun and 20 mM HEPES. After 4 days, cells were washed twice (200xg., 10 min) before being recultured at $2\times10^4$ cells/microwell with IL-2 or IL-4 for 3 days with or without anti-IL-4 receptor antibodies (added 1 h before IL-4 or IL-2). Cells were pulsed with ($^3$H)dThd (1 $\mu$Ci = 37 kBq/well, sp. act: 25 Ci/mmol, CEA, Saclay, France) for the last 16 h of the culture. ($^3$H)dThd uptake was measured by standard liquid scintillation counting techniques after harvesting cells on glass fiber fifters. The results are shown in Figure 1A in which increasing amount of antibodies specific to the human 130 kDa IL-4 receptor decreased the effect IL-4 had on activated T cells as indicated by the lowered intake of tritiated thymidine.

### ● B lymphocyte preparation and biological assays.

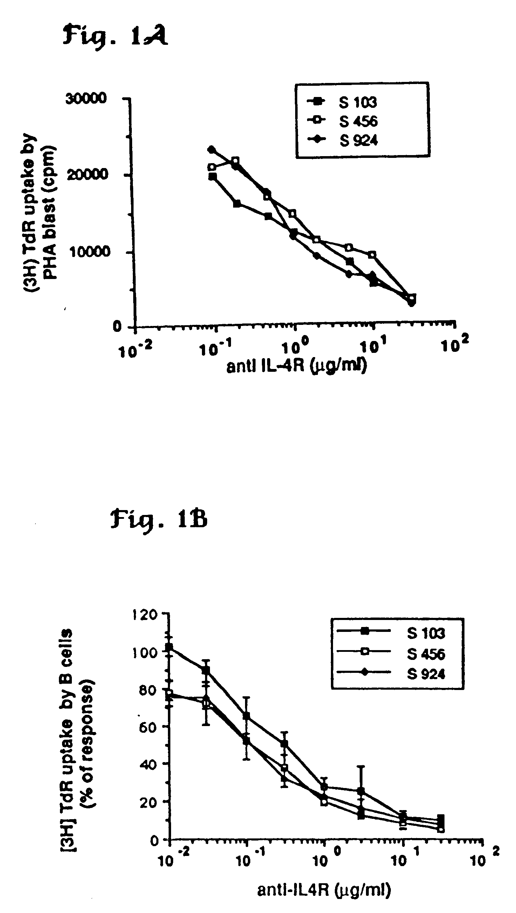

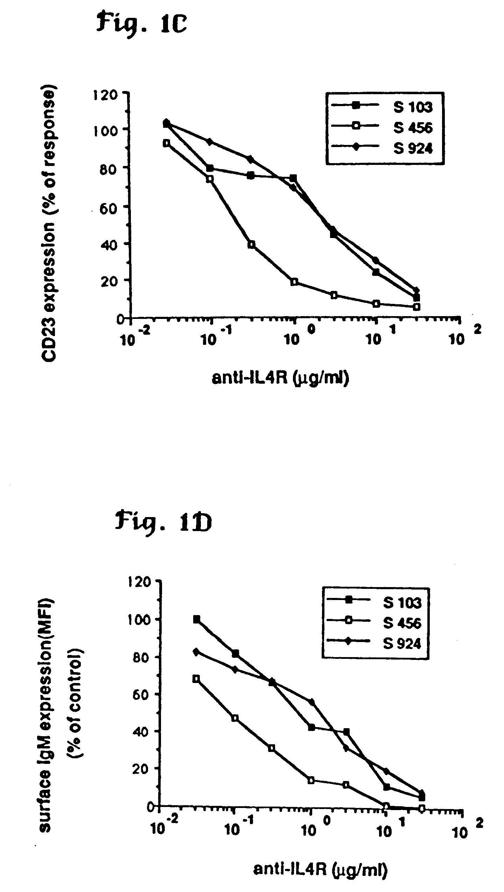

B lymphocytes were purified from tonsils as described previously (Defrance, 1989). Mononuclear cells were rosetted with Sheep red blood cells (SRBC) and contaminating T cells and monocytes were removed using a cocktail of specific mAbs and magnetic beads coated with anti-mouse Ig (Dynabeads, Dynal, Oslo, Norway). Purified B cell preparations contained < 2% T cells and monocytes, as determined by staining with specific anti-CD20, anti-CD2, anti-CD3 and anti-CD14 mAb (Becton-Dickinson, Mountain View, CA). For the study of CD23 expression and surface IgM, $1 \times 10^6$ B cells were cultured in 1 ml complete culture medium for 48 h with or without IL-4. Cells were then washed and stained with anti-CD23 mAb25 (Fc$_\epsilon$RII) or anti-IgM (Becton Dickinson) and FITC-conjugated goat anti-mouse Ig according to standard protocols. Fluorescence staining was analyzed with a FACScan (Becton Dickinson, Sunnyvale, CA). For proliferation assays, B cells (5 $\times10^4$/well) were cultured for 72 h with 5 $\mu$g/ml of insolubilized rabbit anti-IgM antibody (Bio-Rad, Richmond, CA) in the presence of IL-2 or IL-4, and cells were pulsed with ($^3$H)dThd as described for PHA blasts. In all experiments, anti-IL-4 receptor antibodies were preincubated 1 h at 37°C with B cells before adding IL-2 or IL-4 to the culture. The results are shown in Figure 1B in which increasing amount of antibodies specific to the human 130 kDa IL-4 receptor decreased the effect IL-4 had on activated B cells as indicated by the lowered intake of tritiated thymidine; in Figure 1C in which increasing amounts of antibodies specific to the human 130 kDa IL-4 receptor decreased the effect IL-4 had on activated B cells as indicated by the lowered expression of the CD23 receptor; and in Figure 1 D in which increasing amounts of antibodies specific to the human 130 kDa IL-4 receptor decreased the effect IL-4 had on activated B cells as indicated by the lowered expression of the surface IgM on the B cell.

RESULTS

● **Generation of mabs to human IL-4R**.

To produce mAbs specific for the human IL-4 receptor, the hybridoma supernatants derived from mice immunized with the purified soluble IL-4R were screened for their ability to specifically stain the murine fibroblast cell line (C70) stably transfected with the human IL-4R. Ten hybridomas were selected whose supernatant stained C70 but not L cell transfected with the vector alone. Among the ten hybriddoma supernatants, three were able to inhibit IL-4 binding to human cell lines (Jijoye). The ten hybridomas which were cloned, subcloned and secreted mAbs were characterized as of the IgG[1] subtype. Ascites were generated and mAbs secreted by the ten hybridomas purified by HPLC (DEAE).

Table 1 shows that the three inhibitory antibodies (S103, S456, S924) inhibited (90-100%) $^{125}$I-IL-4 binding to activated T cells (PHA blast) or B cells (Jijoye) with a half maximum inhibitory concentrations ranging from 0.7 to 4 nM (S924), 0.9 to 1 nM (S456) and 1.3 to 2.3 nM (S103). These antibodies were also able to completely abolish IL-4 induced biological effects, such as IL-4 induced T or B cell proliferation, and IL-4 induced Fc$_\varepsilon$RII (the low affinity receptor for IgE) or surface IgM expression as is shown in Figures 1A, 1B, 1C and 1D respectively.

● **Characterization of antibody groups by cross-competition for binding to IL4R.**

Based on $^{125}$I-IL-4 binding inhibition and cross-competition between biotinylated S456 and S697; and S697 and S924 and unlabelled mAbs for binding to C70 (Table 2), four groups of antibodies were characterized. The first group included S103, S456; the second, 0361, 0497, 0735; the third, S17, S103, S327; the fourth, S697.

● **Immunoprecipitation and Western blot.**

All these mAbs were further characterized for their ability to immunoprecipitate the $^{125}$I-soluble IL-4R and to recognize in Western blot experiments electroblotted purified IL-4 receptor. Results given in Table 3 show that only the S697 was a good candidate for Western blot detection of the IL-4R, whereas four antibodies namely, S103, S456, S697, and S924 were able to immunoprecipitate the soluble 130 kDa IL-4R.

● **ELISA assay for detection of natural soluble IL-4R.**

Taking advantage of mAbs recognizing non-overlapping epitopes of the IL-4R, an ELISA assay was set up to detect, soluble IL-4 receptor. A cocktail of mAbs was bound to the plastic plate (S17, 0296, 0361, 0497) and after washing, wells were incubated with various concentrations of purified soluble IL-4R. Then, a second biotinylated antibody (S456) was added and S456 binding was revealed with alkaline phosphatase conjugated streptavidin. Figure 2 shows that this sandwich ELISA permitted detection of 10 ng/ml of soluble IL-4 receptor. This ELISA assay might be useful to search for natural soluble IL-4R in human sera.

**Hybridoma Deposit**

Applicants deposited the hybridomas of the present with the Public Health Laboratory Service (PHLS) in Wilshire, UK and they have the following PHLS accession numbers:

| Hybridoma | PHLS Accession Number |
|---|---|
| S103 | 92111901 |
| 0361 | 92111902 |
| S17 | 92111903 |
| 0296 | 92111904 |
| S456 | 92111905 |
| S924 | 92111906 |
| S697 | 92111907 |
| 0497 | 92111908 |
| 0735 | 92111909 |

The Deposits have been made to comply with the requirements of the Budapest Treaty on the Deposit of Microorganisms. Availability of the deposited strain is not to be construed as a license to practice the invention in contravention of the rights granted under the authority of any government in accordance with its patent rights.

EP 0 604 693 A1

Table I

Inhibition of IL-4 binding to cells by mAb anti-IL-4R

| mAb<br>cells | S924 | S456 | S103 |
|---|---|---|---|
| Jijoye cells | 0.7 nM (100%) | 1 nM (100%) | 1.3 nM (90%) |
| PHA blasts | 3. 6 nM (90%) | 0.9 nM (100%) | 2.3 nM (90%)` |

Numbers in brackets represent the percentage of inhibition at the maximum concentration used (10 µg/ml). Number in nM units represent the half maximum inhibitory concentration. All binding experiments were performed with 50 pM of $^{125}$I-IL-4.

EP 0 604 693 A1

## Table II

**Cross competition betwen biotinylated and unlabelled mAb for binding to cell**

| Unlabelled mAb | biot S456c9 | biot S697c1 | biot S924c18 |
|---|---|---|---|
| S176 | NI | NI | NI |
| S103 | I | I | I |
| S456 | I | I | I |
| S924 | I | I | I |
| 0296 | NI | NI | NI |
| 0361 | NI | I | I |
| 0497 | NI | I | I |
| 0735 | NI | I | I |

NI : non inhibitor
I : inhibitor

## TABLE III

### Properties of anti-IL-4R of mAbs

| mAbs | Isotype | Western blot | Immunoprecipitation | Inhibition of IL-4 binding |
|---|---|---|---|---|
| S17 | IgG$_1$ | - | - | - |
| S103 | IgG$_1$ | - | + | + |
| S327 | IgG$_1$ | - | - | - |
| S456 | IgG$_1$ | - | + | + |
| S697 | IgG$_1$ | + | + | - |
| S924 | IgG$_1$ | - | + | + |
| 0296 | IgG$_1$ | - | + | - |
| 0361 | IgG$_1$ | - | - | - |
| 0497 | IgG$_1$ | - | - | - |
| 0735 | IgG$_1$ | - | - | - |

## Claims

1. A monoclonal antibody specific for human 130 kDa interleukin-4 receptor able to inhibit 90-100% of IL-4 binding to activated T cells or B cells.

2. The monoclonal antibody of claim 1 wherein the antibody is able to inhibit 90-100% of the binding of IL-4 to activated T cells or B cells with a half maximum concentration ranging from 0.7 to 4nM.

3. The monoclonal antibody of claim 2 wherein the antibody is produced by hybridoma S924.

4. The monoclonal antibody of claim 1 wherein the antibody is able to inhibit 90-100% of the binding of IL-4 to activated T cells or B cells with a half maximum concentration ranging from 0.9 to 1nM.

5. The monoclonal antibody of claim 4 wherein the antibody is produced by hybridoma S456.

6. The monoclonal antibody of claim 1 wherein the antibody is able to inhibit 90-100% of the binding of IL-4 to activated T cells or B cells with a half maximum concentration ranging from 1.3 to 2.3nM.

7. The monoclonal antibody of claim 6 wherein the antibody is produced by hybridoma S103.

8. A hybridoma capable of producing antibodies specific for a human 130 kDa interleukin-4 receptor selected from the group of hybridomas consisting of : hybridoma S103, hybridoma 0361, hybridoma S17, hybridoma 0296, hybridoma S456, hybridoma S924, hybridoma S697, hybridoma 0497, and hybridoma 0735.

9. An antibody produced by a hybridoma of claim 8 wherein the antibody is humanized.

10. A polypeptide comprising a light chain variable ($V_L$) region wherein the $V_L$ region contains one or more complementary determining regions (CDR) from an antibody produced by a hybridoma of claim 8.

11. A polypeptide comprising a heavy chain variable ($V_H$) region wherein the $V_H$ region contains one or more CDRs from an antibody produced by a hybridoma of claim 8.

12. A binding composition specific for a human 130 kDa interleukin-4 receptor comprising a $V_H$ region and a $V_L$ region wherein the binding composition contains one or more CDRs from an antibody produced by a hybridoma of claim 8.

13. The binding composition of claim 12 wherein the $V_H$ region and $V_L$ region are connected together by a peptide linker.

14. A CDR from an antibody of claim 8.

15. A method of detecting the presence of human interleukin-4 receptor in a sample suspected of containing human interleukin-4 receptor, the method comprising the steps of:
    providing a first monoclonal antibody specific for a first antigenic determinant of human interleukin-4 receptor,.
    providing a second antibody selected from the group consisting of a polyclonal antibody composition specific for human interleukin-4 receptor and a second monoclonal antibody specific for a second antigenic determinant on human interleukin-4 receptor, the second antigenic determinant being different from the first antigenic determinant;
    providing a signal-generating means capable of being operationally associated with either the first monoclonal antibody or the second antibody to produce a signal whose intensity is monotonically related to the amount of either the first monoclonal antibody or the second antibody, respectively;
    attaching either the first monoclonal antibody or the second antibody to a support means to form an antibody-support conjugate;
    contacting the sample with the antibody-support conjugate so that human interleukin-4 receptor in the sample binds to the antibody-support conjugate;
    contacting the first monoclonal antibody operationally associated with the signal generating means with the human interleukin-4 receptor bound to the antibody-support conjugate, when the antibody-support conjugate includes the second antibody; or
    contacting the second antibody operationally associated with the signal-generating means with the human interleukin-4 receptor bound to the antibody-support conjugate, when the antibody-support conjugate includes the first monoclonal antibody; and

measuring the signal generated by the signal-generating means; wherein the monoclonal antibodies are produced by one or more of the hybridomas of claim 8.

16. A kit for detecting the presence of the human interleukin-4 receptor in a sample suspected of containing human interleukin-4 receptor, the kit comprising:

a first monoclonal antibody specific for a first antigenic determinant on human interleukin-4 receptor;

a second antibody selected from the group consisting of a polyclonal antibody composition specific for human interleukin-4 receptor and a second monoclonal antibody specific for a second antigenic determinant on human interleukin-4 receptor, the second antigenic determinant being different from the first antigenic determinant;

a support means; and

a signal generating means; wherein the monoclonal antibodies are produced by one or more of the hybridomas of claim 8.

17. The kit of claim 14 wherein said signal generation means comprises an enzyme operationally associated with said first monoclonal antibody, the enzyme being selected from the group consisting of peroxidase, beta-galactosidase, and alkaline phosphatase.

18. An immunopurification process for extracting human interleukin-4 receptor from a sample containing human interleukin-4 receptor wherein the sample is passed through an immunoadsorbent column comprising a monoclonal antibody specific for human interleukin-4 receptor; wherein the monoclonal antibody is produced from a hybridoma of claim 8.

*Fig. 1A*

*Fig. 1B*

## Fig. 1C

## Fig. 1D

Fig. 2

European Patent
Office

EUROPEAN SEARCH REPORT

Application Number

EP    92 40 3571
Page  1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EUROPEAN JOURNAL OF IMMUNOLOGY vol. 20, no. 8, August 1990, VCH VERLAGSGESELLSCHAFT, DEUTSCHLAND pages 1735 - 1740 MALISZEWSKI CR;SATO TA;VANDEN BOS T;BECKMANN MP;GRABSTEIN KH; 'Induction of B cell activities by interleukin 4 is inhibited by a receptor-specific monoclonal antibody in vitro.' * page 1737, column 1, line 5 - page 1739, column 2, line 42; figure 1 * | 1,2,4,6, 18 | C12P21/08 C07K15/28 C07K13/00 C12N5/20 C07K3/18 G01N33/577 // C12N15/13 |
| X | CELLULAR IMMUNOLOGY vol. 136, August 1991, pages 142 - 154 Ishida H;Yang G;Harada N;Hastings RL;Castle BE;Kastelein R;Miyajima A;Howard M; 'Evaluation of murine interleukin 4 (IL-4) receptor expression using anti-receptor monoclonal antibodies and S1 nuclease protection analyses.' * page 146, line 21 - page 153, line 12; figure 1 * | 1,2,4,6, 18 | |
| X | JOURNAL OF IMMUNOLOGY. vol. 144, no. 11, 1 June 1990, BALTIMORE US pages 4212 - 4217 Beckmann, M.P. et al.; 'Monoclonal antibodies block murine IL-4 receptor function' | 1,2,4,6, 18 | TECHNICAL FIELDS SEARCHED (Int. Cl.5 ) C12P C07K G01N C12N |
| A | * Paragraph "Results"; Figure 1 | 15-17 | |
| X | EP-A-0 367 566 (IMMUNEX CORPORATION, US) 9 May 1990 * example 13 * | 1,2,4,6 | |

-/--

The present search report has been drawn up for all claims

| Place of search | Date of completion of the search | Examiner |
|---|---|---|
| THE HAGUE | 20   1993 | S.A. NAUCHE |

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-8 909 621 (RITTER ET AL., GB) 19 October 1989 * Examples 1-3, claims * | 1,2,4,18 | |
| X | IMMUNOLOGY vol. 65, no. 4, December 1988, pages 617 - 622 Larche M;Lamb JR;O'Hehir RE;Imami-Shita N;Zanders ED;Quint DE;Moqbel R;Ritter MA 'Functional evidence for a monoclonal antibody that binds to the human IL-4 receptor.' * The abstract * | 1,2,4,18 | |
| X | IMMUNOBIOLOGY vol. 183, no. 3/4, 1991, page 22 ENSSLE, K. ET AL.; 'Characterization of monoclonal antibodies specific for the human interleukin-4 receptor' * Abstract I.5 * | 1,2,4,6, 18 | |

TECHNICAL FIELDS SEARCHED (Int. Cl.5 )

The present search report has been drawn up for all claims

| Place of search | Date of completion of the search | Examiner |
|---|---|---|
| THE HAGUE | 20   1993 | S.A. NAUCHE |